# EUROPÄISCHE PATENTSCHRIFT

(11) **EP 1 565 492 B1**
(45) Veröffentlichungstag und Bekanntmachung des Hinweises auf die Patenterteilung: **20.12.2006**
(21) Anmeldenummer: 03775275.5
(22) Anmeldetag: 31.10.2003
(51) Int. Cl.: C07K 16/28, G01N 33/566, G01N 33/564, G01N 33/76

(54) **NACHWEIS VON TSH-REZEPTOR-AUTOANTIKÖRPERN MIT AFFINITÄTSGEREINIGTEN ANTIKÖRPERN**
IDENTIFICATION OF TSH RECEPTOR AUTOANTIBODIES USING AFFINITY-PURIFIED ANTIBODIES
MISE EN EVIDENCE D'AUTO-ANTICORPS RECEPTEURS TSH AVEC DES ANTICORPS SANS AFFINITE

(30) Priorität: 26.11.2002 DE 10255144
(43) Veröffentlichungstag der Anmeldung: 24.08.2005
(73) Patentinhaber: B.R.A.H.M.S Aktiengesellschaft, 16761 Hennigsdorf (DE)
(72) Erfinder: BERGMANN, Andreas, 12351 Berlin (DE); COSTAGLIOLA, Sabine, B-1080 Brussels (BE); VASSART, Gilbert, B-1200 Brussels (BE)
(74) Vertreter: Andrae, Steffen
(86) Internationale Anmeldenummer: PCT/EP2003/012129
(87) Internationale Veröffentlichungsnummer: WO 2004/048415

(56) Entgegenhaltungen:
- WO-A-91/09121
- WO-A-98/20343
- WO-A-98/26294
- MORRIS J C ET AL: "Identification of epitopes and affinity purification of thyroid stimulating auto-antibodies using synthetic human TSH receptor peptides." AUTOIMMUNITY. SWITZERLAND 1994, Bd. 17, Nr. 4, 1994, Seiten 287-299, XP008028212 ISSN: 0891-6934
- BRYANT W P ET AL: "IDENTIFICATION OF THYROID BLOCKING ANTIBODIES AND RECEPTOR EPITOPES IN AUTOIMMUNE HYPOTHYROIDISM BY AFFINITY PURIFICATION USING SYNTHETIC TSH RECEPTOR PEPTIDES" AUTOIMMUNITY, HARWOOD, CHUR, CH, Bd. 22, Nr. 2, 1995, Seiten 69-79, XP000917007 ISSN: 0891-6934
- NAGAYAMA Y ET AL: "BINDING DOMAINS OF STIMULATORY AND INHIBITORY THYROTROPIN (TSH) RECEPTOR AUTOANTIBODIES DETERMINED WITH CHIMERIC TSH-LUTROPIN/CHORIONIC GONADOTROPIN RECEPTORS" JOURNAL OF CLINICAL INVESTIGATION, NEW YORK, NY, US, Bd. 88, Nr. 1, 1. Juli 1991 (1991-07-01), Seiten 336-340, XP002062530 ISSN: 0021-9738
- DALLAS JOHN S ET AL: "A region on the human thyrotropin receptor which can induce antibodies that inhibit thyrotropin-mediated activation of in vitro thyroid cell function also contains a highly immunogenic epitope" JOURNAL OF AUTOIMMUNITY, Bd. 7, Nr. 4, 1994, Seiten 469-483, XP001188293 ISSN: 0896-8411
- MORGENTHALER NILS G ET AL: "Affinity purification and diagnostic use of TSH receptor autoantibodies from human serum." MOLECULAR AND CELLULAR ENDOCRINOLOGY, Bd. 212, Nr. 1-2, 30. Dezember 2003 (2003-12-30), Seiten 73-79, XP001188295 ISSN: 0303-7207 (ISSN print)
- MINICH ET AL CLIN. EXP. IMMUNOL. Bd. 136, 2004, Seiten 129 - 136

## Beschreibung

Die vorliegende Erfindung betrifft verbesserte Rezeptorbindungsassays zur Bestimmung von TSH-Rezeptor-Autoantikörpern (TSHR-Auto-Ab), die bei Schilddrüsen-Autoimmunerkrankungen, insbesondere beim Morbus Basedow, auftreten.

Es ist bekannt, daß zahlreiche Erkrankungen, an denen die Schilddrüse beteiligt ist, Autoimmunerkrankungen sind, bei denen Autoantikörper gegen molekulare Strukturen der Schilddrüse gebildet werden, die im Zusammenhang mit der Erkrankung beginnen, als Autoantigene zu wirken. Die wichtigsten bekannten Auotantigene der Schilddrüse sind dabei Thyreoglobulin (Tg), die Schilddrüsenperoxidase (TPO) und insbesondere der TSH-Rezeptor (TSHR) (vgl. Furmaniak J et al., Autoimmunity 1990, Vol. 7, S. 63-80).

Der TSH-Rezeptor ist ein in der Schilddrüsenmembran lokalisierter Rezeptor, an den das von der Hypophyse ausgeschüttete Hormon TSH (Thyroid-stimulierendes Hormon oder Thyreotropin) bindet und dadurch die Ausschüttung der eigentlichen Schilddrüsenhormone, insbesondere des Thyroxins, auslöst. Der TSH-Rezeptor gehört zur Rezeptor-Familie der G-Protein-gekoppelten Glykoprotein-Rezeptoren mit einer großen aminoterminalen extrazellulären Domäne, zu der auch der LH/CG- und der FSH-Rezeptor gehören. Eine Aufklärung der chemischen Struktur des TSH-Rezeptors, d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur).

Es wird allgemein angenommen, daß bei der als Morbus Basedow (englisch: Graves' disease) bekannten Schilddrüsen-Autoimmunerkrankung stimulierende Autoantikörper eine Rolle spielen, die gegen der TSH-Rezeptor (TSHR) gebildet werden und mit diesem so wechselwirken, daß die Schilddrüse stimuliert wird, was sich als Schilddrüsenüberfunktion (Hyperthyreose) äußert. Es ist jedoch bekannt, dass neben stimulierenden Autoantikörpern in der Zirkulation von menschlichen Patienten auch solche gefunden werden, die gegenteilige oder gar keine klinisch relevanten Wirkungen aufweisen und auch als "blockierende" oder "neutrale" Antikörper bezeichnet werden. Die Bestimmung von Autoantikörpern gegen den TSH-Rezeptor (im folgenden als TSHR-Auto-Ab, oder im Zusammenhang mit Bestimmungen unter Verwendung der von ihr entwickelten Assays in der Terminologie der Anmelderin auch als TRAK, abgekürzt) hat für die Diagnose des Morbus Basedow und anderer Schilddrüsen-Autoimmunerkrankungen eine erhebliche klinische Bedeutung.

Demzufolge werden in der klinischen Diagnostik bereits seit langem routinemäßig Assays zur Bestimmung von gegen den TSHR gebildeten Schilddrüsen-Autoantikörpern eingesetzt.

Bei den älteren Verfahren mußte dabei stets so gearbeitet werden, dass man die zu bestimmenden Autoantikörper mit bovinem TSH (bTSH) um Bindungsstellen einer solubilisierten porcinen TSHR-Präparation konkurrieren ließ und die gebildeten Immunkomplexe für eine Fest-Flüssig-Trennung mit Polyethylenglykol (PEG) ausfällte.

Als modernstes Verfahren zur TSHR-Auto-Ab-Bestimmung ist derzeit das Verfahren anzusehen, das in den veröffentlichten Patentanmeldungen DE 198 01 319.1 A1, der darauf beruhenden WO 99/36782 bzw. im Patent EP 0 975 970 B1 sowie außerdem in der Veröffentlichung Sabine Costagliola et al., Second Generation Assay for Thyrotropin Receptor Antibodies Has Superior Diagnostic Sensitivity for Graves' Disease, in: *J Clin Endocrinol Metab* **84**:90-97, 1999 näher beschrieben ist. Die Lehren dieser Veröffentlichungen praktisch umsetzende Assays sind die unter den Bezeichnung DYNOtest^{®} TRAKhuman^{®} bzw. LUMItest^{®} TRAKhuman^{®} im Handel befindlichen Assays der Anmelderin B.R.A.H.M.S Aktiengesellschaft.

Die genannten Veröffentlichungen DE 198 01 319.1 A1 bzw. die WO 99/36782 enthalten eine ausführliche Diskussion des älteren Standes der Technik und verschiedener mit der Bestimmung von TSHR-Auto-Ab verbundener Schwierigkeiten. Für eine Vertiefung des Verständnisses des technischen Hintergrunds der vorliegenden Erfindung wird daher ausdrücklich auf den Inhalt der genannten Patentanmeldungen sowie der darin diskutierten wissenschaftlichen Veröffentlichungen verwiesen.

Das obige Verfahren gemäß DE 198 01 319.1 A1, der darauf beruhenden WO 99/36782 bzw. dem Patent EP 0 975 970 B1 beruht in erster Linie darauf, dass es bei ihm erstmals möglich war, einen humanen TSHR, und zwar einen rekombinanten humanen TSHR, ohne Funktionalitätsverlust an einer Festphase zu immobilisieren, wodurch die TSHR-Auto-Ab-Bestimmung als heterogener Assay durchführbar wurde und das für die Bestimmung relevante Messsignal direkt an eine Festphase gebunden erhalten wurde.

Als Kompetitor wird auch bei diesem Assay in den Regel ein markiertes bTSH eingesetzt.

Zur Immobilisierung des funktionalen rhTSHR wurde dabei ein monoklonaler Antikörper (BA8) verwendet, der konformative Epitope des TSHR erkannte und nach einem besonderen Verfahren erhalten wird, das beschrieben ist von S.Costagliola und G.Vassart in J.Endocrinol.Invest. 20 (Suppl. to no.5), Abstract 4, (1997) sowie in S.Costagliola et al., Genetic Immunization Against the Human Thyrotropin Receptor Causes Thyroiditis and Allows Production of Monoclonal Antibodies Recognizing the Native Receptor, in: *The Journal of Immunology,* **1998**, 160:1458-1465. Gemäß diesem Verfahren erfolgt eine Immunisierung von Mäusen zum Zwecke der Antikörperbildung nicht mit einem Antigen peptidischer Natur, sondern durch intramuskulare Injektion eines für den hTSHR kodierenden DNA-Plasmidkonstrukts. Es werden auf diese Weise, nach einer geeigneten Selektion, neue monoklonale Antikörper mit hoher Affinität zum nativen hTSHR erhalten, die wenigstens zum Teil, wie BA8, auch konformative Epitope erkennen.

Die Verwendung eines funktionalen immobilisierten humanen TSHR, der in einer durch Waschen im immobilisierten Zustand affinitätsgerenigten Form in die Bestimmungen eingesetzt werden konnte, brachte verschiedene Vorteile und assaytechnische Neuerungen mit sich, von denen insbesondere zu nennen sind:
1. Wenn man die Bestimmung als "Zweischritt-Bestimmung" durchführt, indem man markiertes bTSH in einem nachgeschalteten Schritt mit einem vorher gebildeten und von der ursprünglichen Meßlösung abgetrennten Komplex aus TSHR-Auto-Ab und dem affinitätsgereinigten immobilisierten rhTSHR reagieren läßt, kann sich eine mögliche Anwesenheit von an sich bekannten Anti-bTSH-Ab in der Patientenprobe nicht mehr störend auswirken.
2. Wegen der Verwendung eines humanen Rezeptors kann es zu einer Störung des Assay durch pathologisch hohe hTSH-Konzentrationen, wie sie in einzelnen Patientenproben vorkommen, kommen. Diese Störungen können durch Zusatz von bestimmten kommerziell erhältlichen anti-TSH Antikörpern zur probenhaltigen Meßlösung neutralisiert werden, die das hTSH selektiv binden und nicht mit dem als Kompetitor eingesetzen bTSH kreuzreagieren.
3. Die oben unter 1. aufgeführte neue Erkenntnis ermöglicht es, die Umsetzung mit bTSH im zweiten Assayschritt mit einem exakt standardisierbaren, von Serum freien bTSH-Reagens durchzuführen.
4. Als eine praktische Schwäche des beschriebenen Assays hat es sich erwiesen, dass die Bindungsfähigkeit des im Assay verwendeten humanen rekombinanten TSHR gegenüber dem als Kompetitor verwendeten markierten bovinen TSH relativ schnell verloren geht, und zwar insbesondere dann, wenn der TSHR in der Flüssigphase, z.B. an suspendierte Magnetpartikel gebunden, bereitgestellt oder solubilisiert als Bestandteil homogener Assays eingesetzt wird.

Dadurch, daß das genannte Verfahren eine Bindung der TSHR-Auto-Ab und des markierten Kompetitors bTSH aus der Meßlösung direkt an einen festphasengebundenen, affinitätsgereinigten rekombinaten humanen TSHR gestattete, wird jedoch nicht nur die Messung des erhaltenen Signals gegenüber einem Präzipitationsassay im gewünschten Sinne praktisch vereinfacht, sondern das Assaydesign kann grundsätzlich verändert werden, indem man von einem Einschritt-Assay (eine einzige, durch aufeinanderfolgende Pipettierungen ohne zwischenzeitliche Fest-Flüssig-Trennung erhaltene Meßlösung) zu einem Zweischritt-Assay übergehen kann, bei dem man die Umsetzung des immobilisierten und affinitätsgereinigten gewaschenen TSHR mit der Probe und die Umsetzung mit dem markierten Kompetitor bTSH in zwei aufeinanderfolgenden, durch eine Fest-Füssig-Trennung voneinander abgesetzten Schritten durchführt.

Trotz der Verwendung eines funktionalen humanen TSH-Rezeptors ist das o.g. Verfahren insofern noch einem traditionellen Verfahren ähnlich, als es die Kompetition der zu bestimmenden Autoantikörper mit TSH (in der Regel mit markiertem bovinem TSH) nutzt. Es wurde zwar grundsätzlich diskutiert, dass es an sich möglich sein sollte, in einem kompetitiven Assay statt des markierten bTSH als Kompetitor auch markierte Antikörper, die an den TSH-Rezeptor binden, einzusetzen. Die Zahl von monoklonalen Antikörpern, die unter Einsatz verschiedener Antigene und mittels verschiedener Immunisierungstechniken gegen den vollständigen TSHR bzw. einzelne seiner Abschnitte erzeugt wurden, ist in der Zwischenzeit sehr hoch. Es wurde jedoch noch kein Antikörper beschrieben, der die Eigenschaften stimulierender Antikörper, wie sie als Auslöser des Morbus Basedow postuliert werden, aufweist. In der Praxis gab es daher noch keine Möglichkeit, einen Assay zu schaffen, bei dem ein Kompetitor verwendet wird, der in relevanterer Weise als der Kompetitor bTSH die Anwesenheit und Menge von stimulierenden Autoantikörpern, wie sie für den Morbus Basedow charakteristisch sind, erkennen läßt.

Es ist Aufgabe der vorliegenden Erfindung, einen Rezeptorbindungsassay zur Bestimmung von TSH-Rezeptor-Autoantikörpern zu schaffen, der die Nachteile bekannter kompetitiver Rezeptorbindungsassays des Standes der Technik nicht aufweist und von einer erhöhten klinischen Wertigkeit ist.

Insbesondere ist es ferner Aufgabe der vorliegenden Erfindung, verbesserte Rezeptorbindungsassays zum Nachweis von TSH-Rezeptor-Autoantikörpern so zu gestalten, daß bestimmte Störungen der Messung durch anomale Serumbestandteile auch bei Einschrittverfahren effektiv ausgeschaltet werden und eine optimale Bindung der Reaktionspartner des Bestimmungsverfahrens gewährleistet ist.

Ferner ist es Aufgabe der Erfindung, das Problem des Verlusts der Bindungsfähigkeit des TSHR gegenüber dem Tracer/Kompetitor in der Flüssigphase zu lösen, so dass das Verfahren als übliches automatisiertes Verfahren unter Verwendung von Suspensionen von Magnetpartikeln, an die der TSHR gebunden ist, oder als homogenes Verfahren unter Anwendung der sog. KRYPTOR^{®} Technik duchgeführt werden kann.

Es ist ferner Aufgabe der vorliegenden Erfindung, die zur Durchführung derartiger verbesserter Rezeptorbindungsassays in der klinischen Routinediagnostik erforderlichen Reagenziensätze (Kits) zu schaffen.

Die genannten Aufgaben werden gemäß der vorliegenden Erfindung gelöst durch die Verwendung von affinitätsgereinigten polyklonalen humanen Autoantikörpern gegen den TSH-Rezeptor (TSHR-Auto-Ab) aus Seren von Morbus-Basedow Patienten gemäß Anspruch 1 als spezifisches Bindungsreagens in einem immunologischen Bestimmungsverfahren zum klinischen Nachweis von Autoantikörpern gegen den TSH-Rezeptor (TSHR-Auto-Ab) in einer Probe einer biologischen Flüssigkeit eines auf Morbus Basedow zu untersuchenden Patienten.

Nähere Einzelheiten zu besonders vorteilhaften Ausgestaltungen der verbesserten Rezeptorbindungsassays sind den Unteransprüchen zu entnehmen, insbesondere in Verbindung mit den detaillierteren Erläuterungen in der nachfolgenden Beschreibung.

Die Aufgabe der Schaffung eines Reagenziensatzes zur Verwirklichung der vorliegenden Erfindung wird durch einen bevorzugten Reagenziensatz gemäß Anspruch 7 gelöst, der neben einem funktionalen TSHR als spezifisches Bindungsreagens, insbesondere als markierter Kompetitor, auch eine Präparation von markierten affinitätsgereinigten polyklonalen humanen Autoantikörpern gegen den TSH-Rezeptor (TSHR-Auto-Ab) aus Seren von Morbus-Basedow Patienten gemäß Anspruch 6 enthält.

Die erfindungsgemäßen affinitätsgereinigten Autoantikörper können jedoch auch als spezifischer Binder für einen solubilisierten, ggf. direkt oder indirekt markierten TSHR an eine Festphase immobilisiert eingesetzt werden, z.B. die Wände von Coated Tubes oder Mikropartikel gebunden.

Es erscheint denkbar, anstelle der aus Patientenseren gewonnenen polyklonalen Autoantikörper auch selektierte tierische Antikörper zu verwenden, die mit diesen um Bindungsstellen eines funktionalen humanen TSH-Rezeptors kompetieren, d.h. solche, die die polyklonalen TSHR-Auto-Ab "mimikrieren". Solche tierische Antikörper, insbesondere tierische monoklonale Antikörper, die die gesamte mögliche Autoantikörperpopulation in Patientenseren simulieren, konnten jedoch bisher nicht gefunden und insbesondere nicht sicher identifiziert werden. Bioassays, bei denen untersucht wurde, ob monoklonale Antikörper im Sinne einer Stimulierung der cAMP-Produktion "stimulierende" Antikörper darstellen, ergaben bisher nur negative Resultate. Daraus mußte man schließen, dass die untersuchten Antikörper nicht diejenigen Antikörper repräsentierten, die als pathogene Autoantikörper für den Morbus Basedow anzusehen sind.

In der Einleitung und im nachfolgenden Teil dieser Anmeldung werden die verwendeten Reagenzien bzw. Analyten/Biomoleküle in der Regel mit verschiedenen Abkürzungen gekennzeichnet, die stets - wie in den o.g. Patentanmeldungen - in den folgenden Bedeutungen zu verstehen sind, es sei denn, es ergibt sich aus dem konkreten Zusammenhang für den Fachmann ausnahmsweise etwas anderes. Die Verwendung der speziellen Angaben erfolgt dabei aus Gründen der exakten Beschreibung der durchgeführten Versuche und Messungen, bedeutet jedoch nicht, daß die beschriebenen Ergebnisse und Schlußfolgerungen nur für den beschriebenen Spezialfall gelten. Zahlreiche der vermittelten Informationen sind vielmehr für den Fachmann in ihrer allgemeineren Bedeutung klar erkennbar.

Erläuterungen zu den verwendeten Abkürzungen:
- TSH =: Thyroidstimulierendes Hormon (Thyreotropin). Wird die Abkürzung TSH ohne weitere Zusätze verwendet, handelt es sich nicht um ein bestimmtes Produkt, sondern die Bindung bzw. Funktion des Hormons wird in allgemeiner Form diskutiert.
- bTSH =: Bovines (d.h. aus Rindern gewonnenes) TSH. Präparat, das in herkömmlichen Assays zur Bestimmung von Autoantikörpern gegen den TSH-Rezeptor als Tracer eingesetzt wird.
- hTSH =: Humanes TSH. Kommt im Serum/Plasma Gesunder nur in sehr geringen Konzentrationen 0,2-4 mU/l vor. In Seren von hypothyreoten Patienten kann die hTSH-Konzentration jedoch signifikant erhöht sein. Die durch erhöhte hTSH-Spiegel (von mehr als 20 mU/l) hervorgerufenen Störungen von Autoantikörper-Messungen werden in der o.g. Veröffentlichung WO 99/36782 diskutiert und durch spezielle Maßnahmen neutralisiert.
- TSHR =: Der TSH-Rezeptor, ein in der Schilddrüsenmembran verankerter Glykoproteinrezeptor. Wird die Abkürzung TSHR ohne weitere Zusätze verwendet, handelt es sich nicht um ein bestimmtes Produkt, sondern die Funktion des Rezeptors bzw. seine Bindungsbeteiligung wird in allgemeiner Form diskutiert.
- rhTSHR =: Gentechnisch erzeugtes (rekombinantes) Polypeptid, das die Aminosäuresequenz eines natürlich vorkommenden humanen TSHR wenigstens in einem solchen Ausmaße aufweist, daß es als "funktionaler humaner TSH-Rezeptor" bezeichnet werden kann, was bedeutet, daß es sich bezüglich der Bindung von Autoantikörpern gegen den TSHR bzw. von TSH (bTSH/hTSH) in signifikantem Ausmaß wie der natürlich vorkommende humane TSHR verhält. Wird die Abkürzung rhTSHR ohne weitere Zusätze verwendet, handelt es sich nicht um ein bestimmtes Produkt, d.h. rhTSHR kann für irgendein rekombinantes vollständiges, mehr oder weniger glykosyliertes Polypeptid, eine Teilsequenz einer ausreichenden Länge oder ein gentechnologisch erzeugtes Fusionsprodukt davon stehen (wie es z.B. in der Internationalen Patentanmeldung PCT/EP97/06121 beschrieben wird).
- rhTSHR(imm) =: Selektiv an eine Festphase gebundenes (immobilisiertes) rhTSHR-Präparat. Die Bindung kann wie in der Beschreibung nachfolgend genauer beschrieben - über einen geeigneten Antikörper erfolgen, kann aber im Falle von Fusionsprodukten auch über einen besonderen Peptidrest, z.B. einen Biotinrest, erfolgen. Die Festphase kann die Wand eines Teströhrchens (Coated-Tube- oder CT-Technik) sein, kann aber auch eine geeignete suspendierte Festphase sein. Zur Gewinnung von affinitätsgereinigten polyklonalen Autoantiköreprn wird rhTSHR(imm) an das zur Herstellung der Affinitätssäule verwendete Material gebunden (s. experimenteler Teil).
- Ab =: Antikörper
- TSHR-Auto-Ab =: In biologischen Proben, insbesondere humanem Serum oder Plasma, nachweisbare Autoantikörper gegen den TSH-Rezeptor. Der Nachweis stimulierender derartiger TSHR-Auto-Ab (in der Literatur auch abgekürzt TSI = thyroid stimulating immunglobulins) ist insbesondere für die Diagnose des Morbus Basedow (englisch: Graves' disease) von Bedeutung. Eine weitere übliche Abkürzung ist TRAK.
- Anti-hTSHR-mAb =: Monoklonaler Antikörper, der an rhTSHR bindet. Ohne weitere Erläuterungen kann er, was sein Bindungsverhalten angeht, sequentieller Natur sein, wie z.B. in der älteren Anmeldung DE 196 51 093.7 beschrieben wird, er kann jedoch auch konformativer Natur sein, wie z.B. BA8.
- Anti-bTSH-Ab =: In humanen Seren bzw. Plasma vorkommende Antikörper ungeklärten Ursprungs, die mit bTSH unter Bildung von Immunkomplexen reagieren und damit die Bindung von bTSH an die Assaykomponenten bzw. das erhaltene Meßergebnis beeinflussen (vgl. Y.Ochi et al., Acta Endocrinologica (Copenh) 1989, 120: 773-777; S.Sakata et al., J.Endocrinol. Invest. 14: 123-130, 1991; T. Inui, Thyroid, Vol.6: 295-299, 1996).

Das immunologische Bestimmungsverfahren (Ligandenbindungstest), bei dem die neuen selektiven Bindungsreagenzien gemäß der vorliegenden Erfindung verwendet wird, kann grundsätzlich von jeder geeigneten Art sein, wie sie dem Fachmann aus der Literatur und z.B. den o.g. Anmeldungen bekannt ist.

Wird bei der Bestimmung mit einem an einer Festphase immobilisierten TSHR gearbeitet, können als eine solche Festphase eine Kunststoffoberfläche, insbesondere in Form der Wände von Kunststoff-Teströhrchen für die CT-Technik, Mikropartikel, Magnetpartikel, Filter, Polymergelmaterialien und andere bekannte Festphasenträger eingesetzt werden. Das Assaydesign kann auch so angepaßt werden, daß die Bestimmung an bekannten automatisierten Systemen durchführbar ist (vgl. z.B. Elecsys-System der Firma Boehringer Mannheim oder ACS 180-System von Chiron).

Durch die Verwendung der erfindungsgemäßen Reagenzien werden zahlreiche Vorteile erhalten, auf die im Anschluss an den experimentellen Teil noch einmal eingegangen wird. Es ist jedoch darauf hinzuweisen, dass ein besonderer Vorteil eine Ausgestaltung als Einschritt-Verfahren ist, da aufgrund der homologen Natur der als Bindungsreagens eingesetzten polyklonalen Autoantikörper Störungen, die auf hTSH oder auf bTSH bindende Antikörper in der zu vermessenden Probe zurückzuführen wären, keine Bedeutung haben.

Ein weiterer Vorteil liegt darin, dass die Markierung der polyklonalen Antikörper (bzw. der diese mimikrierenden tierischen Antikörper) nach bekannten Techniken mit nahezu jedem beliebigen bekannten Markierungsreagens möglich ist, ohne dass die Schwierigkeiten zu überwinden sind, auf die man bei der Markierung von TSH stößt. Die Markierung kann dabei auch ein Teil eines an sich bekannten Paares von Detektionsmarkern für ein Verfahren sein, bei dem die spezifischen Bindungsreagenzien (solubilisierter TSHR; Kompetitor in Form von Autoantikörpern) in der flüssigen Reaktionsmischung dispergiert vorliegen, wobei an den Antikörper eine erste Markierungskomponente gebunden ist, die Teil eines auf Fluoreszenz- oder. Chemilumineszenz-Löschung oder -Verstärkung beruhenden Markierungssystems ist, und daß die zweite Markierungskomponente dieses Markierungssystems an einen zweiten, zur indirekten Markierung des solubilisierten TSHR verwendeten nicht kompetierenden Antikörper oder direkt an den solubilisierten TSHR gebunden ist, so dass nach Bindung der markierten Antiköprer an den TSHR ein messbares Signal, z.B. eine Fluoreszenzsignal erzeugt wird, das eine Detektion der gebildeten Sandwichkomplexe in der Messlösung ermöglicht. Für diese Verfahrensvariante ist die längere Bindungsfähigkeit des TSHR gegenüber der erfindungsgemäßen Autoantikörper-Präparation, und zwar verglichen mit der Bindungsfähigkeit gegenüber TSH, ein praktisch äußerst wichtiger Vorteil.

Nachfolgend wird die vorliegende Erfindung in ihren verschiedenen Aspekten anhand von konkreten Ausführungsbeispielen und Versuchsergebnissen unter Bezugnahme auf fünf Figuren noch näher erläutert. Auf die allgemeinen Erläuterungen im Zusammenhang mit den beschriebenen Versuchen wird ausdrücklich als Teil der Offenbarung der vorliegenden Erfindung verwiesen.

Dabei zeigen die Figuren in Form verschiedener Diagramme:

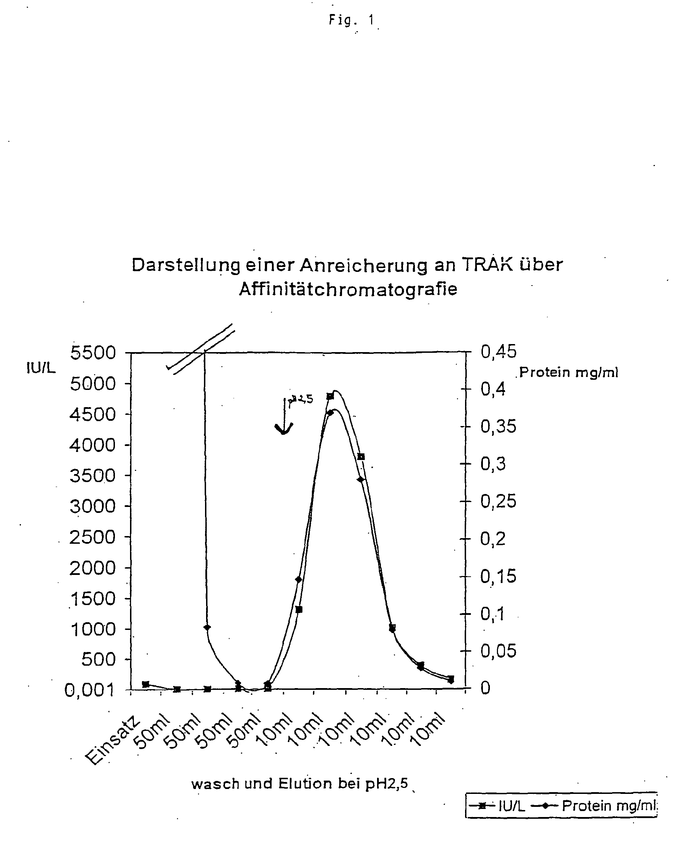

- Figur 1:: Die im Rahmen der Affinitätsreinigung von Autoantikörpern aus Seren von Morbus Basedow Patienten beim Waschen (mit 3 x 50ml PBS) und der nachfolgenden Eluierung (mit jeweils 10 ml Zitronensäure, pH 2,5) erhaltenen Elutionsprofile. Die mit Zitronensäure eluierte Antikörperfraktion ist chemisch homogen.

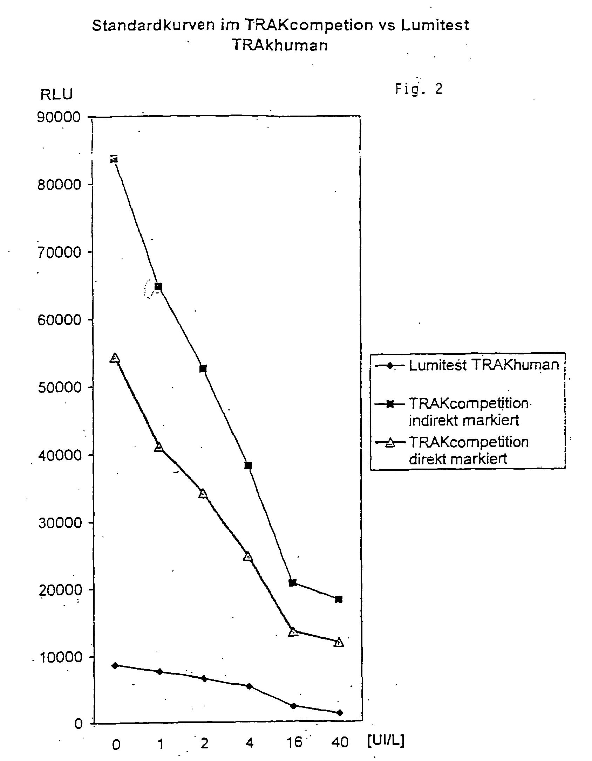

- Figur 2:: Die Standardkurven für die Messung von TSHR-Auto-Ab unter Verwendung von mit einem Akridiniumester direkt bzw. indirekt markierten affinitätsgereinigten polyklonalen humanen Autoantikörpern, gegenübergestellt der Standardkurve eines LUMItest^{®} TRAKhuman^{®} der Anmelderin bei gleichem Maßstab für die Signalstärke (RLU).

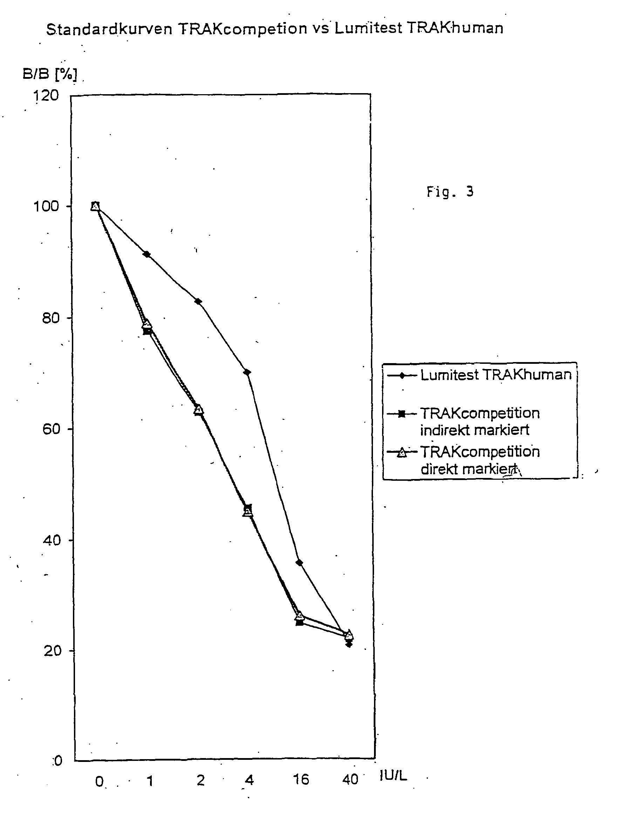

- Figur 3:: Die Standardkurven für die Messung von TSHR-Auto-Ab unter Verwendung von mit einem Akridiniumester direkt bzw. indirekt markierten affinitätsgereinigten polyklonalen humanen Autoantikörpern, gegenübergestellt der Standardkurve eine LUMItest^{®} TRAKhuman^{®} der Anmelderin, bezogen auf die prozentuale Bindung (B/B₀).

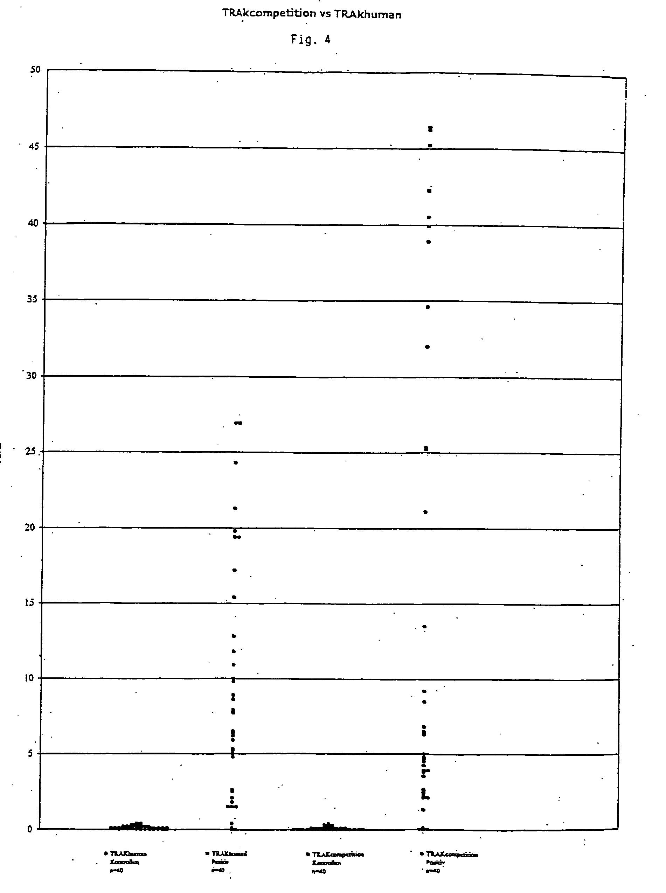

- Figur 4:: Die Ergebnisse der Messung von Patientenseren (40 Kontrollseren; 40 Seren von Morbus Basedow-Patienten) mit Hilfe des LUMItest^{®} TRAKhuman^{®} der Anmelderin (linke Spalten) und des Tests (TRAKkompetition) unter Verwendung markierter affinitätsgereinigter polyklonaler Autoantikörper aus Patientenseren (rechte Spalten). Es ist eine auf die größere Signalstärke zurück zu führende größere Spreizung der Skala und damit unterscheidungskräftigere Messung zu erkennen.

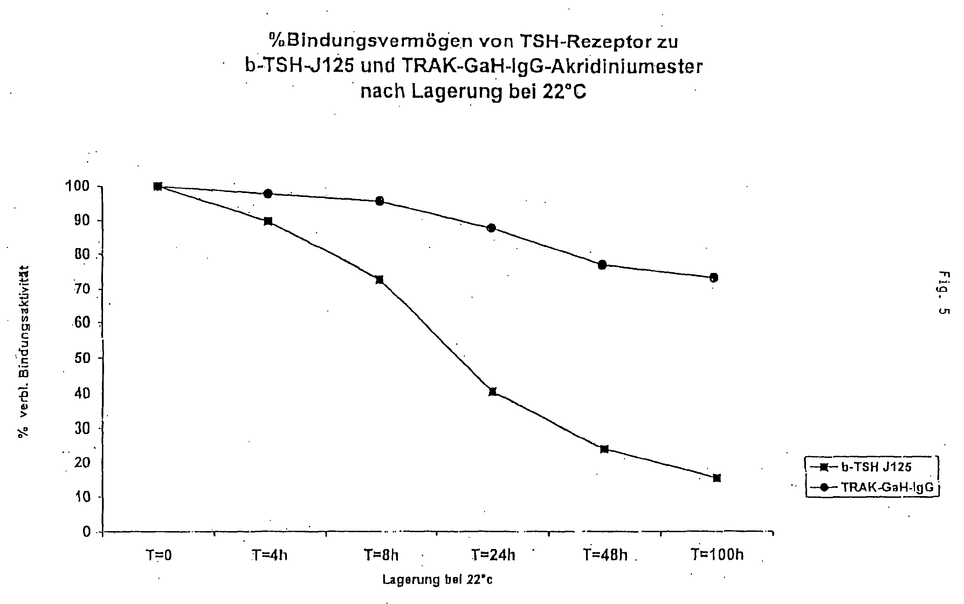

- Figur 5:: Veränderung des Bindungsvermögens einer TSHR-Präparation gegenüber ¹²⁵I-bTSH bzw. mit einem Akridiniumester markierten affinitätsgereinigten polyklonalen Autoanitkörpern bei eine Lagerung bei 22°C.

In der nachfolgenden Beschreibung von Herstellungs- und Meßexperimenten wird - soweit möglich - auf in der vorveröffentlichten Literatur zu findenden Angaben verwiesen, wenn an sich bekannte Techniken und Materialien verwendet wurden:

### 1. Materialien

### 1 Herstellung einer Präparation affinitätsgereinigter polyklonaler humaner Autoantikörper aus Seren von Morbus Basedow Patienten

### 1.1. Herstellung einer Affinitätssäule mit einem gebundenen TSHR

20 mg des bekannten monoklonalen konformationellen Antikörpers BA8 in affinitätsgereinigter Form (vgl. Sabine Costagliola et al., Second Generation Assay for Thyrotropin Receptor Antibodies Has Superior Diagnostic Sensitivity for Graves' Disease, in: *J Clin Endocrinol Metab* **84**:90-97, 1999; S.91, Selection of moAb by FACS; bzw. S.Costagliola et al., Genetic Immunization Against the Human Thyrotropin Receptor Causes Thyroiditis and Allows Production of Monoclonal Antibodies Recognizing the Native Recpetor, in: *The Journal of Immunology,* **1998**, 160:1458-1465), 2 mg/ml in phosphatgepufferter Salzlösung (PBS), wurden mit 50 mg NaIO₄ versetzt, 20 min bei Raumtemperatur inkubiert und anschließend mit Hilfe von NAP25-Säulen (Pharmacia), äquilibriert in PBS, entsalzt. Die gewonnene Proteinfraktion (14 ml) wurde mit PBS gewaschen, mit Carbolink-Material (5 ml Gel; Fa. Pierce) vermischt und unter leichtem Schütteln 20 h bei 4°C inkubiert.

Die erhaltene Suspension wurde auf einen Glasfilter gegeben und mit 200 ml PBS gewaschen. Das gewaschene Carbolink-Material mit dem daran gebunden Antikörper BA8 wurde dann in einer Lösung eines rekombinanten humanen TSHR (vgl. Sabine Costagliola et al., Second Generation Assay for Thyrotropin Receptor Antibodies Has Superior Diagnostic Sensitivity for Graves' Disease, in: *J Clin Endocrinol Metab* **84:**90-97, 1999; S.91, Preparation of hTSH-R) resuspendiert (Volumen 250 ml, Extrakt aus 6 x 10⁹ K562 Zellen) und für 10 h bei 4°C inkubiert.

Das resultierende Festphasenmaterial wurde über einen Glasfilter 3 mal mit 100 ml PBS gewaschen.

### 1.2. Affinitätsreinigung von Autoantikörpern aus Seren von Morbus Basedow Patienten

Das gemäß 1.1 erhaltene gewaschene Festphasenmaterial wurde in 400 ml eines Pools von Seren von Morbus Basedow Patienten (mittlere Konzentration 190 U/l, Total 75 U) suspendiert und für 10 h bei 4°C unter leichtem Schütteln inkubiert.

Danach wurde das Festphasenmaterial in eine am Boden mit einer Glasfritte verschlossene Glassäule (2 x 10 cm) gefüllt und dreimal mit jeweils 50 ml PBS gewschen. Dabei betrug die Durchflussgeschwindigkeit 1 ml/min. Nach dem letzen Waschen war das Eluat proteinfrei.

Anschließend erfolgte die Elution der über den immobilisierten TSHR an das Festphasenmaterial gebundenen Autoantikörper (in der Figur als TRAK abgekürzt) unter Verwendung von 10 ml Portionen 50-mM Zitronensäure bei einer Durchflußgeschwindigkeit von 1 ml/min. Der Säulenausfluss wurde in Fraktionen von 1 ml aufgefangen.

Die erhaltenen 1 ml-Fraktionen wurden unter Verwendung des DYNOtest^{®} TRAKhuman^{®} der Anmelderin auf die Anwesenheit von Anti-TSHR-Antikörpern geprüft, und die Proteinkonzentrationen wurden mittels der BCA-Methode ermittelt. Die als identisch anzusehenden Kurvenzüge deuten darauf hin, dass Proteinmenge und Antikörperaktivität einander entsprachen und eine als chemisch homogen zu bezeichnende Antikörperfraktion gewonnen worden war.

Die Ergebnisse sind in Figur 1 und der nachfolgenden Tabelle wiedergegeben.

**Tabelle 1**

| Volumen | TRAKhuman [IU/L] | TRAK total [IU] | Protein [mg/ml] | Protein total [mg] | spezifische Aktivität mg Protein/1TRAK IU |
|---|---|---|---|---|---|
| 400 ml (Spenderseren) | 190 | 76 | 78 | 31200 | 411 |
| 10 ml (Eluat) | 4795 | 47,95 | 0,37 | 3,7 | 0,076 |

Die Ergebnisse zeigen, dass eine hoch aufgereinigte Autoantikörperpräparation mit hoher Antikörperaktivität (mehr als 1 IU/mg Protein) hergestellt worden war. Eine solche affinitätsgereinigte Präparation ist nicht mit Produkten vergleichbar, wie sie in der ferneren Vergangenheit unter Verwendung von Präparationen aus Schilddrüsenmembranen angereichert wurden, z.B. gemäß Rosalind Brown et al., Partial Purification and Characterization of Thyrotropin Binding Inhibitory Immunoglobulins from Normal Human Plasma, *J Clin Endocrinol Metab* **56**: 156-162 (1983).

### 1.3. Markierung der affinitätsgereinigten polyklonalen Autoantikörper aus Morbus Basedow Seren

### 1.3.1 Mit Akridiniumester direkt chemilumineszenz-markierter Antikörper

30 µl (100 µg) des wie unter 1.2. beschrieben affinitätsgereinigten Autoantikörpers wurden mit 70 µl 1 M Na-PO₄ (pH 7,2) neutralisiert und mit 10 µl NHS-Akridiniumester (Fa. Behringwerke AG, Marburg; vgl. EP 0 257 541 B1; 1 mg/ml in Acetonitril) vermischt und für 10 min bei Raumtemperatur inkubiert. Anschließend wurde überschüssiger Akridinium-Aktivester mit 10 µl 0,1 Glycin/NaOH (pH 7,2) abgesättigt, und ungebundener Akridiniumester über GelfiltrationsHPLC an einer SW 300-Säule (Millipore) präpariert. Alle Fraktionen des Säulenausflusses mit einer UV-Absorption bei 280 nm und 368 nm wurden gesammelt. Die gepoolte Proteinfraktion wurde bis zur weiteren Verwendung in Rezeptorbindungsassays bei -80°C gelagert.

### 1.3.2. Indirekt Markierung über einen Ziegen anti-Human Antikörper

Alternativ zu 1.3.1. wurden die affinitätsgereinigten Autoantikörper in einem molaren Verhältnis von 1:1 mit Akridiniumester-markierten Ziegen anti-Human IgG (Grade 2, Scantibodies) umgesetzt (100 µg Autoantikörper plus 100 µg Ziegen Anti-Human Akridiniumester in 10 ml PBS, 1% BSA). Nach eines zweistündigen Inkubation wurde das Materail auf 20 ng (ca. 0,25 mU Antikörper) pro 100 µl in einem Puffer verdünnt, der aus PBS, 1% BSA und 10% Humanserum bestand.

Die beiden direkt (1.3.1.) bzw indirekt (1.3.2.) markierten Autoantikörper werden dann in den nachfolgend beschriebenen Messungen von Kontrollen und Patientenseren als Tracer verwendet.

### 1.4 Standards

Als Standards wurden die Standards des LUMItest^{®} TRAK der Anmelderin verwendet.

### 2. Assay-Durchführung: Pipettier- und Inkubations-Protokolle

Zur Evaluierung der neuen markierten Tracerpräparationen auf der Basis affinitätsgereinigter Autoantikörper aus Seren von Morbus Basedow Patienten wurden Bestimmungen mit der folgenden Folge der Pipettier- und Inkubationsschritte durchgeführt:
- 1.: Pipettiere 100 µl Probe (Patientenserum, Standardserum oder Nullstandardserum) in mit immobilisiertem rhTSHR beschichtete Teströhrchen aus dem LUMItest^{®}TRAKhuman^{®} der Anmelderin.
- 2.: Pipettiere dazu direkt (1.3.1) oder indirekt (1.3.2) markierte affinitätsgereinigte Autoantikörper, jeweils in 100 µl.
- 3.: Inkubiere 2 h unter Schütteln (300 U/min) bei Raumtemperatur auf einem Heidolph-Kreisschüttler.
- 4.: Pipettiere 1 ml Waschlösung (PBS).
- 5.: Dekantiere.
- 6.: Wiederhole Schritt 4 und 5 je drei mal.
- 7.: Messe den an der Wand des Teströhrchens verbliebenen Akridiniumetser durch Auslösung der Lumineszenzreaktion als Chemilumineszenz-Lichtausbeute mit einem geeigneten Luminometer, z.B. Berthold LB 952 T/16.
- 8.: Bestimmung von TSHR-Auto-Ab in Patientenseren

Die Ergebnisse der Messung von 40 Kontrollseren und 40 Patientenseren mit den beiden unterschiedlich markierten Autoantikörperfraktionen sind in den Figuren 2 und 3 gezeigt.

Es ist zu erkennen, dass ein wesentlich höheres spezifisches Signal als im LUMItest^{®} TRAKhuman^{®} erhalten wird, während im Hinblick af die Diagnose des Morbus Basedow eine vergleichbare bzw. sogar höhere Sensitivität und Spezifität erhalten wird.

Überraschender Weise wurde ferner festgestellt, dass der als spezifischer Bindungspartner in die Bestimmungen eingesetzte immobilisierte rekombinante humane TSH-Rezeptor genau wie ein solubilisierter TSHR seine Bindungsfähigkeit gegenüber den markierten Autoantikörpern, und damit seine Eignung als Reagens für die Antikörperbestimmung, deutlich länger behielt als bei Verwendung von markiertem bTSH als Tracer, was einen erheblichen praktischen Vorteil darstellt.

Da der Assay ein sog. homologer Assay ist, bei dem Tracer und zu bestimmender Ligand im wesentlichen identisch sind, sind Störungen durch eine Beeinflussung der Messung aufgrund der Anwesenheit von hTSH nicht möglich - dieses wirkt sich auf die Tracerbindung und Antikörperbindung identisch aus. Die Tracerbindung wurde bei hTSH-Konzentrationen bis zu 500 U/L getstet, und es wurde kein Störeinfluss aufgrund der Anwesenheit von hTSH festgestellt.

### 3. Untersuchung der Stabilität des Bindungsverhaltens von TSHR-Präparationen gegenüber verschiedenen Tracern

Der rekombinante humane TSHR wurde, wie beschrieben, mit 2% Triton in HEPES, 10% Glycerin, 10 mM EDTA, 0,5% BSA, pH 7,4, aus K 562-Zellen extrahiert und als Extrakt bei 22°C gelagert.

Nach den in Figur 5 angegebenen Intervallen wurde jewels 1 Aliquot der TSHR-Suspension auf Polystyrolröhrchen, die mit dem monoklonalen Antikörper (BA8) vorbeschichtet waren, gegeben und für 1 h bei 4°C auf einem Kreisschüttler (Heidolph) bei 300 U/min inkubiert. Das Inkubationsvolumen betrug 300 µl. Anschließend wurde das Röhrchen mit HEPES Puffer, pH 7,4, 0,5% BSA, befüllt, der Röhrcheninhalt abgesaugt, und dieser Schritt wurde 2 mal wiederholt.

Der gebundene TSHR wurde anschließend durch Vakuumtrocknung stabilisiert. Die getrockneten Röhrchen wurden schließlich mit 200 µl Tracer-Lösung aus dem TRAKhuman^{®} der Anmelderin, die radioaktiv markiertes bTSH enthielt, oder mit 200 µl der wie oben unter 1.3.2 beschrieben hergestellten indirekt markierten affinitätsgereinigten Autoantikörperpräparation für 2 h bei 22°C unter Schütteln (300 U/min) inkubiert.

Anschließend erfolgte jeweils die Entfernung des ungebundenen markierten Materials durch Waschen der Röhrchen mit PBS (Zugabe von 2 ml PBS/Absaugen, 3 mal wiederholt). Die auf der Röhrchenoberfläche zurrückgebliebene Markierung wurde zum Schluß in einem Gamma-Counter bzw. einem Luminometer vermessen.

Die Ergebnisse sind in Figur 5 gezeigt. Es ist deutlich zu erkennen, dass mit zunehmender Lagerungsdauer immer weniger an markiertem bTSH-Tracer gebunden wurde und bei den Proben nach 100 h Lagerung die bTSH-Bindung auf etwa 20% des Anfangswerts erniedrigt war, während die Bindung der markierten Autoantikörperfraktion im gleichen Zeitraum nur wenig abgenommen hatte. Da eine Abnahme der Bindungsfähigkeit in einem homologen Assay Tracer und Analyt gleichermaßen betrifft, während das bei der Bindungsfähigkeit gegenüber einem bTSH-Tracer im Vergleich mit dem Analyten nicht zutreffen muss, bedeuten die Befunde, dass die Messgenauigkeit sehr viel weniger Zeiteinflüssen unterliegt als bei dem bekannten Assay.

### 4. Identifizierung/Selektion von tierischen Antikörpern gegen den hTSHR, die mit Autoantikörpern aus Seren von Morbus Basedow Patienten um Bindungsstellen eines funktionalen rekombinaten humanen TSH-R kompetieren

Aufgrund seiner Natur als homologer Assay erlaubt ein Assay unter Verwendung einer Präparation von markierten affinitätsgereinigten polyklonalen Autoantikörpern aus Morbus Basedow Seren als Kompetitor eine Selektion solcher tierischer Antikörper, insbesondere monoklonaler Antikörper, die tatsächlich mit Morbus Basedow Autoantikörpern kompetieren und daher ggf. solche mit stimulierender Wirkung sind, wie sie bisher noch nicht beschrieben wurden.

Unter Anwendung der Technik der genetischen Immunisierung gemäß S.Costagliola et al., Genetic Immunization Against the Human Thyrotropin Receptor Causes Thyroiditis and Allows Production of Monoclonal Antibodies Recognizing the Native Receptor, in: *The Journal of Immunology,* **1998**, 160:1458-1465, wurde eine große Anzahl von tierischen monoklonalen Antikörpern gegen den TSH-R erzeugt.

Bei einer Prüfung im oben beschriebenen homologen Assay zeigte sich, dass von 3000 getesteten Klonen nur 7 in der Lage waren, mit den markierten affinitätsgereinigten polyklonalen Autoantikörpern aus Morbus Basedow Seren zu kompetieren. Die monoklonalen Antikörper dieser 7 Klone zeigten sämtlich im Bioassay eine Stimulation der cAMP-Produktion und sind daher in der Lage, die bei Morbus Basedow auftretenden Autoantikörper gegen den TSH-R zu mimkrieren. Damit ist gezeigt, dass - wenn auch mit einer anscheinend eher geringen Häufigkeit - monoklonale Antikörper tatsächlich erzeugbar sind, die in ihrem Bindungsverhalten den natürlichen Autoantikörpern entsprechen und daher allein oder in ihrer Mischung oder in einer Mischung bzw. Kombination mit affinitätsgereinigten Autoantikörpern als spezifische Bindungsreagenzien (Kompetitor/Tracer; Festphasenkomponente; sekundärer Kompetitor) für die Morbus Basedow Diagnostik, z.B. anstelle der aus Patientenseren durch Affinitätsreinigung gewinnbaren Antikörper, eingesetzt werden können.

## Patentansprüche

1. Verwendung von affinitätsgereinigten polyklonalen humanen Autoantikörpern gegen den TSH-Rezeptor (TSHR-Auto-Ab),
die bis zur biochemischen Homogenität gereinigt sind und eine spezifische Aktivität von mindestens 1 IU/mg Protein (Human-Immunglobulin) besitzen
und die erhalten wurden durch eine affinitätschromatographische Reinigung, bei der die Autoantikörper aus einem Pool von Seren von Morbus Basedow Patienten an ein Affinitätsmaterial mit einem daran immobilisierten funktionalen humanen rekombinanten TSH Rezeptor gebunden, proteinfrei gewaschen und anschließend von dem Affinitätsmaterial eluiert wurden,
als spezifisches Bindungsreagens in einem immunologischen Bestimmungsverfahren zum klinischen Nachweis von Autoantikörpern gegen den TSH-Rezeptor (TSHR-Auto-Ab) in einer Probe einer biologischen Flüssigkeit eines auf Morbus Basedow zu untersuchenden Patienten.

2. Verwendung nach Anspruch 1, wobei man die affinitätsgereinigten polyklonalen humanen Autoantikörper gegen den TSH-Rezeptor (TSHR-Auto-Ab) aus Seren von Morbus-Basedow Patienten in direkt oder indirekt markierter Form in einem kompetitiven immunologischen Bestimmungsverfahren als Kompetitoren für die in einer Probe eines Patienten zu bestimmenden Autoantikörper gegen den humanen TSH-Rezeptor verwendet.

3. Verwendung nach einem der Ansprüche 1 oder 2, wobei das immunologische Bestimmungsverfahren ein kompetitives Verfahren ist, bei dem man als spezifischen Bindungspartner für die zu bestimmenden Autoantikörper und für die als Kompetitoren verwendeten affinitätsgereinigten polyklonalen humanen Seren von Morbus-Basedow Patienten eine Präparation eines funktionalen rekombinanten humanen TSH-Rezeptors in festphasengebundener Form oder in solubilisierter Form verwendet.

4. Verwendung nach einem der Ansprüche 1 bis 3, wobei man die affinitätsgereinigten polyklonalen humanen Autoantikörpern gegen den TSH-Rezeptor (TSHR-Auto-Ab) aus Seren von Morbus-Basedow Patienten in festphasengebundener Form einsetzt und als spezifischen Bindungspartner einen solubilisierten funktionalen humanen rekombinaten TSH-Rezeptor in direkt oder indirekt markierter Form verwendet.

5. Verwendung nach einem der Ansprüche 1 bis 3, **dadurch gekennzeichnet, dass** man die affinitätsgereinigten polyklonalen humanen Autoantikörpern gegen den TSH-Rezeptor (TSHR-Auto-Ab) aus Seren von Morbus-Basedow Patienten in mit einem Radioisotop, mit einem Chemilumineszenzlabel, mit einem Fluoreszenzlabel oder in mit Hilfe eines entsprechend markierten tierischen anti-human IgG markierter Form verwendet.

6. Affinitätsgereinigte polyklonale humane Autoantikörper gegen den TSH-Rezeptor (TSHR-Auto-Ab) aus Seren von Morbus-Basedow Patienten, die unter Verwendung eines Affinitätsmaterials mit einem daran gebundenen funktionalen humanen rekombinanten TSH Rezeptor bis zur biochemischen Homogenität affinitätsgereinigt wurden und eine spezifische Aktivität von mindestens 1 IU/mg Protein (Human-Immunglobulin) besitzen, in markierter Form.

7. Reagenziensatz für die klinische Diagnostik zur immunologischen Bestimmung von Autoantikörpern gegen den TSH-Rezeptor in Patientenseren, der neben den üblichen Komponenten wie insbesondere Verdünnungs- und/oder Pufferlösungen, Standards und Nullstandard sowie ggf. Kontrollseren als zwei weitere Komponenten einen solubilisierten oder festphasengebundenen humanen TSH-Rezeptor sowie eine markierte Präparation von affinitätsgereinigten polyklonalen humanen Autoantikörpern gegen den TSH-Rezeptor (TSHR-Auto-Ab) aus Seren von Morbus-Basedow Patienten gemäß Anspruch 6 enthält.

## Claims

1. Use of affinity-purified polyclonal human autoantibodies against the TSH receptor (TSHR-Auto-Ab)
which are purified to biochemical homogeneity and have a specific activity of at least 1 IU/mg of protein (human immunoglobulin), and
which were obtained by purification by affinity chromatography, in which the autoantibodies from a pool of sera of Graves' disease patients were bound to an affinity material having a functional human recombinant TSH receptor bound thereto, washed protein-free and then eluted from the affinity material
as a specific binding reagent in an immunological assay method for clinical determination of autoantibodies against the TSH receptor (TSHR-Auto-Ab) in a sample of a biological fluid of a patient to be investigated for Graves' disease.

2. Use according to claim 1, wherein the affinity-purified polyclonal human autoantibodies against the TSH receptor (TSHR-Auto-Ab) from sera of Graves' disease patients are used in directly or indirectly labelled form in a competitive immunological assay method as competitors for the autoantibodies against the human TSH receptor which are to be determined in a sample of a patient.

3. Use according to any of claims 1 or 2, wherein the immunological assay method is a competitive method in which a preparation of a functional recombinant human TSH receptor in a form bound to a solid phase or in solubilized form is used as a specific binding partner for the autoantibodies to be determined and for the affinity-purified polyclonal human autoantibodies against the TSH receptor (TSHR-Auto-Ab) obtained from sera of Graves' disease patients and used as competitors.

4. Use according to any of claims 1 to 3, wherein the affinity-purified polyclonal human autoantibodies against the TSH receptor (TSHR-Auto-Ab) from sera of Graves' disease patients are used in a form bound to the solid phase, and a solubilized functional human recombinant TSH receptor in directly or indirectly labelled form is used as a specific binding partner.

5. Use according to any of claims 1 to 3, **characterized in that** the affinity-purified polyclonal human autoantibodies against the TSH receptor (TSHR-Auto-Ab) from sera of Graves' disease patients are used in a form labelled with a radio isotope, a chemiluminescent label or a fluorescent label or with the aid of a correspondingly labelled animal anti-human IgG.

6. Affinity-purified polyclonal human autoantibodies against the TSH receptor (TSHR-Auto-Ab) from sera of Graves' disease patients, which are affinity-purified to biochemical homogeneity with the aid of an affinity material having a functional human recombinant TSH receptor bound thereto and have a specific activity of at least 1 IU/mg of protein (human immunoglobulin), in labelled form.

7. Reagent kit for clinical diagnostics for the immunological determination of autoantibodies against the TSH receptor in patient sera, which, in addition to the customary components, such as, in particular, dilution and/or buffer solutions, standards and zero standard and optionally control sera, contains, as two further components, a human TSH receptor which is solubilized or bound to a solid phase and a labelled preparation of affinity-purified polyclonal human autoantibodies against the TSH receptor (TSHR-Auto-Ab) from sera of Graves' disease patients according to claim 6.

## Revendications

1. Utilisation d'auto-anticorps humains polyclonaux purifiés par affinité, contre le récepteur TSH (TSHR-auto-Ac), qui sont purifiés jusqu'à homogénéité biochimique et qui possèdent une activité spécifique d'au moins 1 UI/mg de protéine (immunoglobuline humaine) et qui sont obtenus par purification par chromatographie d'affinité, dans laquelle les auto-anticorps d'un ensemble de sérums de patients avec maladie de Basedow, sont liés sur un matériau d'affinité avec un récepteur TSH recombiné humain fonctionnel immobilisé, lavés jusqu'à absence de protéine, et ensuite élués du matériau d'affinité, comme réactifs spécifiques de liaison dans un procédé de détermination immunologique pour la détection clinique d'auto-anticorps contre le récepteur TSH (TSHR-auto-Ac) dans un échantillon de fluide biologique d'un patient examiné pour la maladie de Basedow.

2. Utilisation selon la revendication 1, où l'on utilise l'auto-anticorps humain polyclonal purifié par affinité, contre le récepteur TSH (TSHR-auto-Ac), provenant de sérums de patients avec maladie de Basedow, sous forme marquée directe ou indirecte, dans un procédé de détermination immunologique compétitif, comme compétiteur pour les auto-anticorps contre le récepteur TSH humain, à déterminer dans un échantillon d'un patient.

3. Utilisation selon la revendication 1 ou 2, où le procédé de détermination immunologique est un procédé compétitif, dans lequel on utilise comme partenaire spécifique de liaison pour l'auto-anticorps à déterminer et pour l'auto-anticorps humain polyclonal purifié par affinité, contre le récepteur TSH (TSHR-auto-Ac), provenant de sérums de patients avec maladie de Basedow, utilisé comme compétiteur, une préparation d'un récepteur TSH humain recombiné, fonctionnel, sous formée liée à une phase solide ou sous forme solubilisée.

4. Utilisation selon l'une des revendications 1 à 3, où l'on met en oeuvre l'auto-anticorps humain polyclonal purifié par affinité, contre le récepteur TSH (TSHR-auto-Ac), provenant de sérums de patients avec maladie de Basedow, sous une forme liée à une phase solide et comme partenaire spécifique de liaison, un récepteur TSH humain recombiné, fonctionnel, sous formée marquée directe ou indirecte.

5. Utilisation selon l'une des revendications 1 à 3, **caractérisée en ce que** l'on utilise l'auto-anticorps humain polyclonal purifié par affinité, contre le récepteur TSH (TSHR-auto-Ac), provenant de sérums de patients avec maladie de Basedow, sous une forme marquée avec un radioisotope, un marqueur chimioluminescent, un marqueur fluorescent ou avec un IgG anti-humain animal marqué de manière appropriée.

6. Auto-anticorps humain polyclonal purifié par affinité, contre le récepteur TSH (TSHR-auto-Ac), provenant de sérums de patients avec maladie de Basedow, qui a été purifié jusqu'à homogénéité biochimique en utilisant un matériau d'affinité avec un récepteur TSH recombiné, humain, fonctionnel, y lié, et qui possède une activité spécifique d'au moins 1 UI/mg de protéine (immunoglobuline humaine), sous forme marquée.

7. Kit de réactifs pour le diagnostic clinique pour la détermination immunologique d'auto-anticorps contre le récepteur TSH dans le sérum de patient, qui contient en plus des composants usuels, comme en particulier, des solutions de dilution et/ou tampon, les standards et les standards nuls ainsi que le cas échéant, des sérums témoins, comme deux autres composants, un récepteur TSH humain solubilisé ou lié à une phase solide, ainsi qu'une préparation marquée d'auto-anticorps humain polyclonal purifié par affinité, contre le récepteur TSH (TSHR-auto-Ac), provenant de sérums de patients avec maladie de Basedow, selon la revendication 6.
